# EUROPÄISCHE PATENTSCHRIFT

(11) **EP 1 648 351 B1**
(45) Veröffentlichungstag und Bekanntmachung des Hinweises auf die Patenterteilung: **25.02.2009**
(21) Anmeldenummer: 03739938.3
(22) Anmeldetag: 22.07.2003
(51) Int. Cl.: A61F 2/44

(54) **ZWISCHENWIRBELIMPLANTAT MIT TEMPORÄREN BLOCKIERMITTELN**
INTERVERTEBRAL IMPLANT COMPRISING TEMPORARY BLOCKING MEANS
IMPLANT INTERVERTEBRAL COMPORTANT DES ELEMENTS DE BLOCAGE TEMPORAIRES

(43) Veröffentlichungstag der Anmeldung: 26.04.2006
(73) Patentinhaber: Synthes GmbH, 4436 Oberdorf (CH)
(72) Erfinder: LECHMANN, Beat, CH-2544 Bettlach (CH); BÜRKI, Roger, CH-4710 Balsthal (CH); FRIGG, Robert, CH-2544 Bettlach (CH)
(74) Vertreter: Lusuardi, Werther
(86) Internationale Anmeldenummer: PCT/CH2003/000496
(87) Internationale Veröffentlichungsnummer: WO 2005/007041

(56) Entgegenhaltungen:
- FR-A- 2 737 656
- US-A- 5 314 477

## Beschreibung

Die Erfindung bezieht sich auf ein Zwischenwirbelimplantat, insbesondere künstliche Bandscheibe, gemäss dem Oberbegriff des Patentanspruchs 1.

Nach Entfernung einer beschädigten, natürlichen Bandscheibe oder eines beschädigten Nukleus pulposus einer Bandscheibe werden heute Implantate oder Prothesen in den Zwischenwirbelraum zweier benachbarter Wirbelkörper eingebracht. Das Ziel der Implantation solcher Implantate ist es, wieder möglichst natürliche Zustände herbeizuführen, d.h. insbesondere die ursprüngliche Bandscheibenhöhe und damit den ursprünglichen Abstand zwischen den beiden benachbarten Wirbelkörpern wiederherzustellen. Ferner sollen Bewegungen der benachbarten Wirbelkörper relativ zueinander möglichst ohne Behinderung in ihrer natürlichen Art ausführbar sein. Hierzu ist die Erhaltung der Bewegungsmöglichkeiten bei einer Vorwärts/Rückwärtsneigung, d.h. Flexion und Extension der Wirbelkörper sowie bei einer lateralen Beugung der Wirbelkörper innerhalb der natürlichen Grenzen wesentlich. Die natürlichen Bänder und Muskeln entlang der Wirbelsäule werden im wesentlichen intakt gelassen, so dass diese die Bewegungen eines mechanischen Bandscheibenersatzes weiter stabilisieren.

Ein solches Zwischenwirbelimplantat ist aus der US 5,556,431 BÜTTNER bekannt. Dieses bekannte Implantat umfasst eine untere und eine obere Deckplatte, deren Aussenfläche an die Deck- respektive Grundplatte der angrenzenden Wirbelkörper zu Anlage bringbar sind, sowie ein zwischen den Deckplatten angeordnetes Gelenk. Dieses Gelenk besteht im wesentlichen aus einem sphärisch konvexen Gelenkteil und zwei mit den Deckplatten verbundenen, komplementären Gelenkschalen als zweite Gelenkteile, so dass die Deckplatten relativ zueinander polyaxial schwenkbar sind. Nachteilig an diesem bekannten Zwischenwirbelimplantat ist, dass zwar die zwei Deckplatten je mit einem Gelenkteil verbunden sind, aber die Gelenkteile nicht zusammengehalten werden.

Aus der WO 2004/054478 AEBI ist ein Zwischenwirbelimplantat bekannt, welches ein zwischen Deckplatten angeordnetes Gelenk, sowie Mittel zur Blockierung der Beweglichkeit der Deckplatten relativ zum Gelenk umfasst.

Ein weiteres Zwischenwirbelimplantat, welches ein zwischen Deckplatten angeordnetes Gelenk umfasst ist aus US 5,314,477 MARNAY bekannt.

Der Erfindung liegt die Aufgabe zugrunde, ein Zwischenwirbelimplantat zu schaffen, das ein rotativ bewegbares Gelenk aufweist, dessen Teile in einem relativ zueinander blockierten Zustand vormontierbar sind und in-situ deblockierbar sind.

Die Erfindung löst die gestellte Aufgabe mit einem Zwischenwirbelimplantat, insbesondere einer künstlichen Bandscheibe, welches die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank des erfindungsgemässen Zwischenwirbelimplantates
- niedrige Herstellungskosten erreichbar sind;
- das Implantat vor dessen Implantation vormontierbar ist, so dass dieses nicht vom Chirurgen zusammengesetzt werden muss und keine Fehlmanipulationen auftreten können;
- das Implantat vor und während der Implantation nicht auseinanderfallen kann, respektive keine Teile verloren gehen können;
- die Gelenkteile mit den Deckplatten verbunden sind, so dass sie sich nicht verschieben und das umliegende Gewebe zerstören können;
- eine einfache Ausgestaltung der Mittel zur Aufnahme der Blockiermittel möglich ist;
- eine Entfernung der Blockiermittel möglich ist auch wenn das Distraktionsinstrument noch mit dem Zwischenwirbelimplantat verbunden ist;
- durch Anbringen von Blockiermitteln auf beiden Seiten der Mittelebene eine stabilere Fixation der zwei Teil erreichbar ist; und
- die Fixation bezüglich der Mittelebene symmetrisch ist, wodurch die Handhabung der chirurgischen Instrumente in-situ wesentlich vereinfacht wird.

In einer bevorzugten Ausführungsform sind die Blockiermittel einstückig ausgebildet. Die dadurch erreichbare einfache Ausgestaltung gestattet eine einfache Handhabung bei der Montage sowie bei der Demontage der Blockiermittel am Zwischenwirbelimplantat.

In einer anderen Ausführungsform sind die Blockiermittel derart ausgestaltet, dass sie eine selbsttätige Fixation der zwei Teile gestatten. Dies ist beispielsweise durch mindestens teilweise elastische Ausgestaltung derselben oder durch Keilwirkung bei der Montage der Blockiermittel erreichbar. Damit ist der Vorteil erreichbar, dass die Fixation der zwei Teile ohne äusseren Einfluss, wie beispielsweise manuelles Zutun möglich ist.

In einer weiteren Ausführungsform münden die Nuten in die ventrale Seitenfläche des jeweiligen Teiles. Damit ist erreichbar, dass die Blockiermittel nach der Implantation des Zwischenwirbelimplantates einfach herausgezogen werden können. Zudem erfolgt das Herausziehen der Blockiermittel auf der selben Achse wie die Implantation des Zwischenwirbelimplantates, so dass kein zusätzliches Präparieren im Zwischenwirbelraum notwendig ist.

In einer anderen Ausführungsform verjüngt sich die quer zur Mittelebene stehende Querschnittsfläche der Nuten gegen die obere, respektive untere Oberfläche der zwei Teile, so dass die Nuten beispielsweise als Schwalbenschwanzführungen ausgebildet sind, und in beiden zur Zentralachse parallelen Richtungen Kräfte aufnehmen können.

In wiederum einer anderen Ausführungsform sind die Nuten gekrümmt und weisen bogenförmige Längsachsen auf, wobei die Abstände der Längsachsen zur Mittelebene gegen die ventrale Seitenflächen zunehmen. Hierdurch ist erreichbar, dass das Zwischenwirbelimplantat vor der Implantation mit greifzangenartigen Blockiermitteln blockiert und zusammengehalten werden kann, so dass die Teile sich während der Implantation nicht relativ zueinander bewegen können und keines der Teile verloren gehen kann.

In einer weiteren Ausführungsform umfassen die Blockiermittel einen zur Zentralachse parallel angeordneten Verbindungssteg und endständig an diesem je einen Schenkel oder Quersteg. Im ersten Fall können die Blockiermittel beispielsweise als U-förmige Klammern mit elastischen Schenkeln und/oder einem elastischen Verbindungssteg ausgestaltet sein. Im zweiten Fall ist beispielsweise eine Doppel-T förmige Ausgestaltung möglich. Diese Ausgestaltungen haben den Vorteil einer einfachen Herstellung und somit niedrigen Kosten der Blockiermittel. Ferner ist die Handhabung der Blockiermittel einfach. Komplexere Ausgestaltungen der Blockiermittel wären auch anfälliger auf Brüche oder Klemmen.

In einer anderen Ausführungsform sind die Blockiermittel zangenartig ausgestaltet und umfassen zwei um ein Drehgelenk relativ zueinander bewegbare, in die Nuten einführbare Backen, wobei die Drehachse des Drehgelenkes vorzugsweise parallel zur Zentralachse verläuft. Damit ist eine einfache Herstellung und somit niedrige Kosten der Blockiermittel erreichbar. Ferner ist ein Öffnen und Schliessen der Blockiermittel ohne Verkanten möglich.

In einer weiteren Ausführungsform umfasst das konvexe Gelenkteil eine sphärische, erste Gelenkfläche und die Gelenkschale eine dazu komplementäre, zweite Gelenkfläche. Durch die sphärische Ausgestaltung der Gelenkflächen ist der Vorteil erreichbar, dass die beiden Teile polyaxial relativ zueinander schwenkbar sind.

Die Abmessungen der Gelenkflächen sind je nach Anwendung verschieden, wobei in den verschiedenen Ausführungsformen der Radius der ersten Gelenkfläche des konvexen sphärischen Gelenkteiles zwischen 3 mm und 25 mm, vorzugsweise zwischen 4 mm und 20 mm beträgt.

In wiederum einer weiteren Ausführungsform bestehen das konvexe Gelenkteil und die Gelenkschale aus einer Metall - Kunststoff Materialpaarung. Damit sind die Vorteile erreichbar, dass
- bereits bewährte Kombinationen von Gelenkersatzmaterialien, wie beispielsweise ein hochvernetztes Polyethylen (X-UHMWPE) und eine Kobalt-Chrom Legierung einsetzbar sind;
- geringe Reibungskräfte bei der relativen Verschiebung der Gleitflächen erreichbar sind; und
- eine Dämpfung für axiale Stossbelastungen erreichbar ist.

In einer anderen Ausführungsform sind die Gelenkflächen A;B mit Titankarbid oder amorphem Kohlenstoff (ADLC) beschichtet, wodurch eine erhebliche Verringerung des Reibungskoeffizienten erreichbar ist.

In wiederum einer anderen Ausführungsform sind die zwei Teile an den Appositionsflächen mit Titan beschichtet.

In einer weiteren Ausführungsform sind die Appositionflächen konvex ausgebildet und an die natürliche Deck- respektive Grundfläche der angrenzenden Wirbelkörper angepasst.

In wiederum einer weiteren Ausführungsform sind die zwei Teile an ihren Appositionsflächen mit makroskopischen Strukturen versehen, welche vorzugsweise als Erhebungen ausgebildet sind.

Diese Erhebungen können in verschiedenen Ausführungsformen:
- pyramidenartige Erhebungen umfassen;
- mindestens eine zur Mittelebene symmetrische, keilartige Rippe umfassen, welche sich entlang einer antero-posterioren Geraden auf der betreffenden Appositionsfläche steht; oder
- zur Mittelebene symmetrisch angeordnete, sägezahnartige Zacken umfassen.

Durch diese Ausgestaltungen der makroskopischen Strukturen sind die Vorteile erreichbar, dass einerseits Drehmomente um eine die Appositionsflächen schneidende Drehachse besser von den Wirbelkörpern auf das Zwischenwirbelimplantat übertragen werden können und andererseits die Fläche, woran die Knochen anwachsen können erhöht werden kann. Die Zacken weisen den Vorteil auf, dass sie besser in die Endplatten der angrenzenden Wirbelkörper eindringen können. Die pyramidenförmigen Erhöhungen weisen vorzugsweise ein Volumen V zwischen 0,12 mm³ und 1,4 mm³ auf.

In einer anderen Ausführungsform sind die Erhebungen mindestens teilweise mit Hydroxylapatit oder mit einem bi-phasischen Hydroylapatit-Trikalziumphosphat Gemisch beschichtet, wodurch der Vorteil erreichbar ist, dass beide erwähnte Materialien vollständig in den Knochen integriert oder sogar durch neues, natürliches Knochengewebe ersetzt werden.

Die Blockiermittel können in ihren verschiedenen Ausführungsarten ein maximales Volumen von 12 cm³, vorzugsweise von 6 cm³ aufweisen.

In wiederum einer anderen Ausführungsform umfasst das Zwischenwirbelimplantat eine sterile Verpackung, so dass das vormontierte und mittels der Blockiermittel fixierte Zwischenwirbelimplantat vom Chirurgen ohne vorgängige Manipulation in-situ implantierbar ist.

Die Erfindung und Weiterbildungen der Erfindung werden im folgenden anhand der teilweise schematischen Darstellungen eines Ausführungsbeispieles noch näher erläutert.

Es zeigen:
Fig. 1 eine perspektivische Ansicht einer Ausführungsform des Zwischenwirbelimplantates mit montierten Blockiermitteln;
Fig. 2 eine Explosionsdarstellung der in Fig. 1 dargestellten Ausführungsform des Zwischenwirbelimplantates mit Blockiermitteln;
Fig. 3 einen medio-lateralen Schnitt durch die in den Fig. 1 und 2 dargestellte Ausführungsform des Zwischenwirbelimplantates mit Blockiermitteln;
Fig. 4 eine perspektivische Darstellung einer anderen Ausführungsform des Zwischenwirbelimplantates mit in die Nuten eingeführten Blockiermitteln;
Fig. 5 eine Explosionsdarstellung der in Fig. 4 dargestellten Ausführungsform des Zwischenwirbelimplantates mit Blockiermitteln;
Fig. 6 einen zur medio-lateralen Ebene parallelen Schnitt durch die Nuten und Blockiermittel der in den Fig. 4 und 5 dargestellten Ausführungsform des Zwischenwirbelimplantates mit Blockiermitteln;
Fig. 7 einen medio-lateralen Schnitt durch die in den Fig. 4 und 5 dargestellte Ausführungsform des Zwischenwirbelimplantates mit Blockiermitteln;
Fig. 8 eine perspektivische Darstellung einer weiteren Ausführungsform des Zwischenwirbelimplantates mit in die Nuten eingeführten Blockiermitteln;
Fig. 9 eine perspektivische Darstellung des unteren Teils der in Fig. 8 dargestellten Ausführungsform des Zwischenwirbelimplantates mit aus den Nuten ausgefahrenen Blockiermitteln;
Fig. 10 eine Aufsicht auf das untere Teil der in den Fig. 8 und 9 dargestellten Ausführungsform des Zwischenwirbelimplantes mit in die Nuten eingeführten Blockiermitteln;
Fig. 11 eine perspektivische Ansicht des oberen Teils einer Ausführungsform des Zwischenwirbelimplantates mit Blockiermitteln;
Fig. 12 eine Ansicht von lateral auf das obere Teil der in Fig. 5 dargestellten Ausführungsform des Zwischenwirbelimplantates mit Blockiermitteln;
Fig. 13 eine Ansicht von lateral auf das obere Teil einer weiteren Ausführungsform des Zwischenwirbelimplantates mit Blockiermitteln; und
Fig. 14 eine Ansicht von dorsal auf das obere Teil der in Fig. 7 dargestellten Ausführungsform des Zwischenwirbelimplantates mit Blockiermitteln.

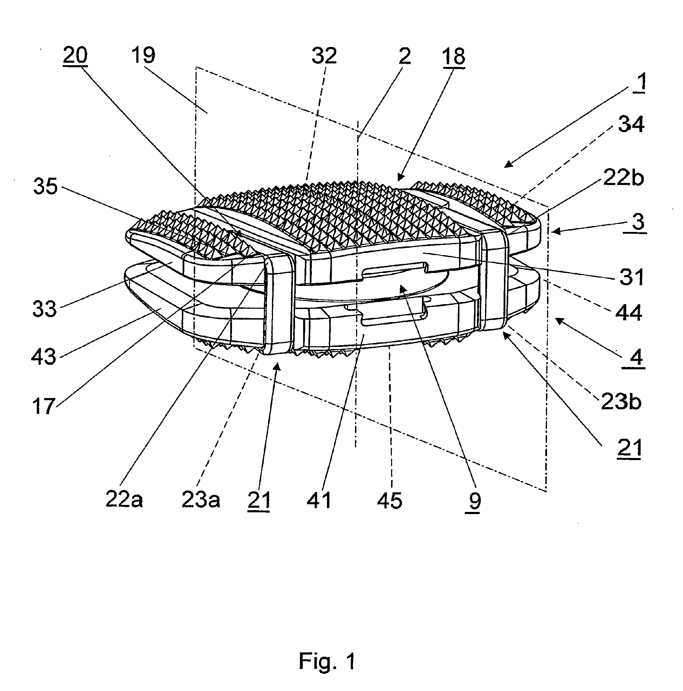

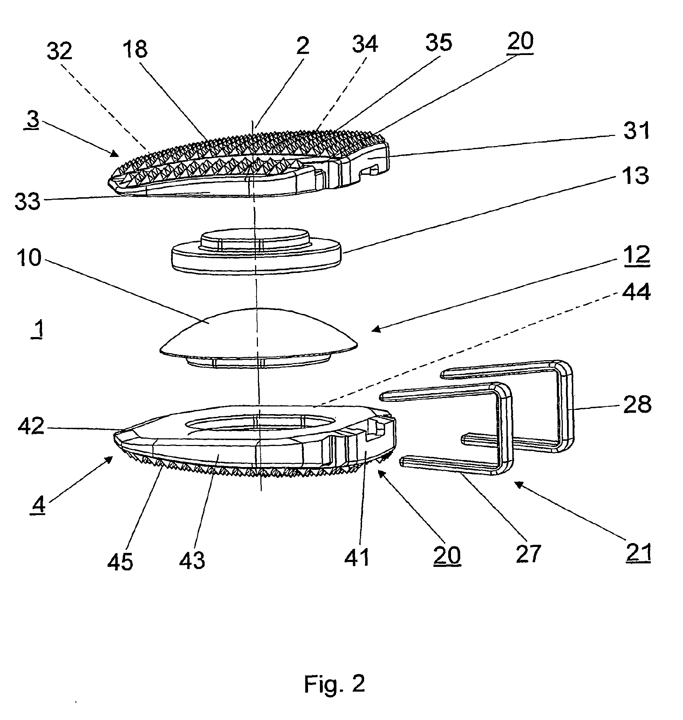

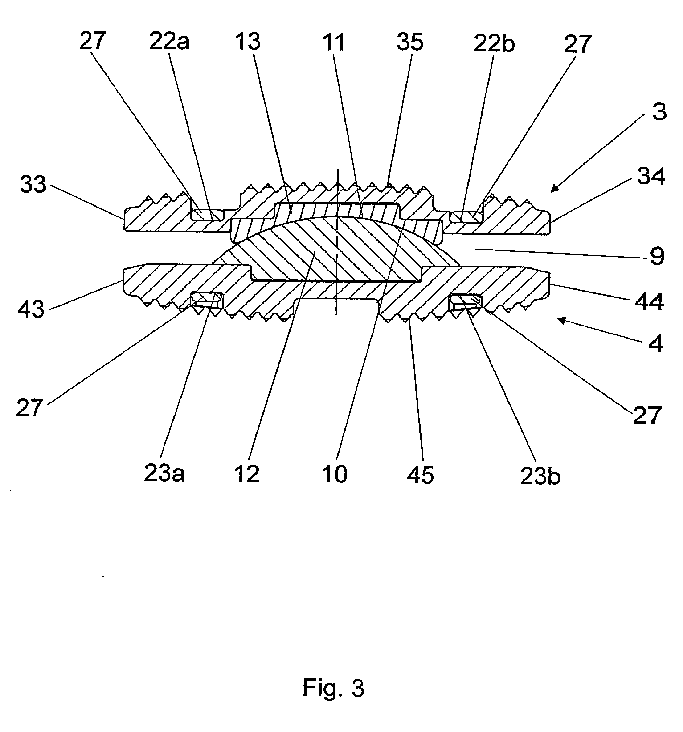

Die in den Fig. 1 bis 3 dargestellte Ausführungsform umfasst im wesentlichen ein oberes Teil 3 mit einer die Zentralachse 2 schneidenden, oberen Appositionsfläche 35 zur Anlage an die Grundplatte des oberhalb angrenzenden Wirbelkörpers, ein unteres Teil 4 mit einer die Zentralachse 2 schneidenden, unteren Appositionsfläche 45 zur Anlage an die Deckplatte des unterhalb angrenzenden Wirbelkörpers und ein zwischen den zwei Teilen 3;4 angeordnetes Gelenk 9 zur gelenkigen Verbindung der zwei Teile 3;4. Das Gelenk 9 ist zweiteilig ausgebildet, wobei das konvexe Gelenkteil 12 sphärisch ausgestaltet ist, eine erste Gelenkfläche 10 aufweist und mit dem unteren Teil 4 verbunden ist, und die zum konvexen Gelenkteil 12 komplementär ausgestaltete Gelenkschale 13 eine zweite Gelenkfläche 11 aufweist und mit dem oberen Teil 3 verbunden ist. Das obere Teil 3 und die Gelenkschale 13 sind in der hier dargestellten Ausführungsform zweiteilig, so dass das obere Teil 3 beispielsweise aus Titan oder einer Kobalt-Chrom Legierung hergestellt und die Gelenkschale 13 beispielsweise aus einem Kunststoff hergestellt sein kann.

Jedes der zwei Teile 3;4 umfasst eine ventrale Seitenfläche 31;41, eine dorsale Seitenfläche 32;42, sowie quer dazu je zwei laterale Seitenflächen 33;34;43;44. Mittig zwischen den lateralen Seitenflächen 33;34;43;44 liegt die Mittelebene 19, wobei die Zentralachse 2 bei nicht artikuliertem Gelenk 9 in dieser Mittelebene 19 liegt. Die Erhebungen 18 sind hier als pyramidenartige Erhebungen 17 ausgestaltet.

Ferner umfassen die zwei Teile 3;4 Mittel 20 zur Aufnahme von zwei temporär befestigbaren Blockiermitteln 21, welche zur lösbaren Fixation der zwei Teile 3;4 relativ zueinander geeignet sind. Die Mittel 20 umfassen hier am oberen Teil 3 und am unteren Teil 4 je zwei Nuten 22;23, deren Längsachsen 24;25 zur Mittelebene 19 des Zwischenwirbelimplantates 1 symmetrisch beabstandet sind. Dabei sind je eine obere und eine untere Nute 22a;23a mit gleichem Abstand zur Mittelebene 19 auf der linken Seite der Mittelebene 19 und je eine obere und eine untere Nute 22b;23b mit gleichem Abstand zur Mittelebene 19 auf der rechten Seite der Mittelebene 19 angeordnet sind. Die Nuten 22;23 münden in die jeweiligen ventralen Seitenflächen 31;41 so dass die Blockiermittel 21 von ventral in die Nuten 22;23 einführbar oder herausziehbar sind. Ferner sind die Nuten 22;23 gegen die jeweilige Appositionsfläche 35;45 offen.

Jedes der zwei Blockiermittel 21 ist als Klammer 26 mit zwei Schenkeln 27 und einem die Schenkel 27 verbindenden Steg 28 ausgestaltet, derart, dass auf einer Seite der Mittelebene 19 ein Schenkel 27 einer Klammer 26 in eine obere Nute 22a und der andere Schenkel 27 derselben Klammer 26 in die untere Nute 23a einführbar sind, so dass die Klammer 26 parallel zur Mittelebene 19 angeordnet ist. Analog sind die Schenkel 27 einer zweiten Klammer 26 in die Nuten 22b;23b auf der anderen Seite der Mittelebene 19 einführbar. Die Klammern 26 sind parallel zur Zentralachse 2 elastisch deformierbar, so dass die Schenkel 27 nach ihrer Einführung in die Nuten 22;23 gespreizt werden und die Blockiermittel 21 durch elastische Klemmwirkung an den zwei Teilen 3;4 lösbar befestigbar sind.

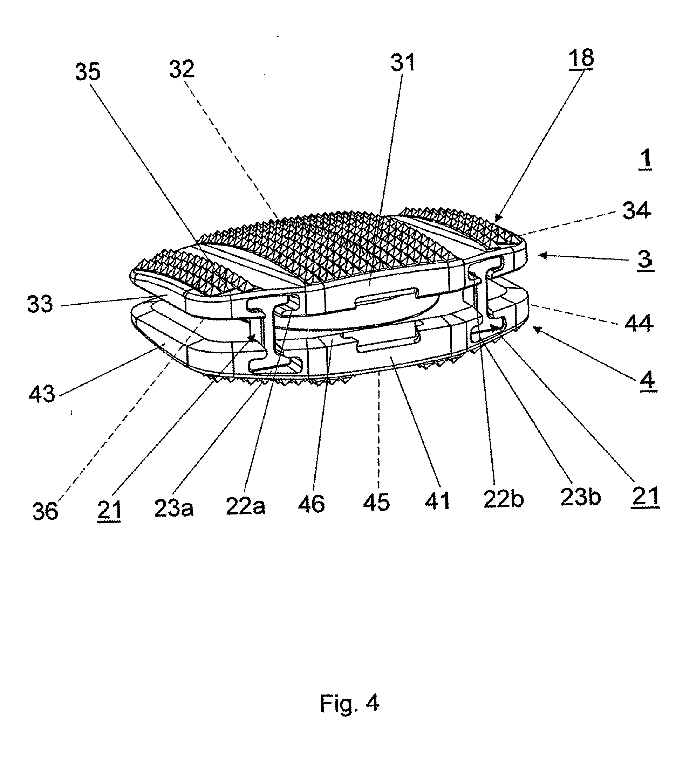

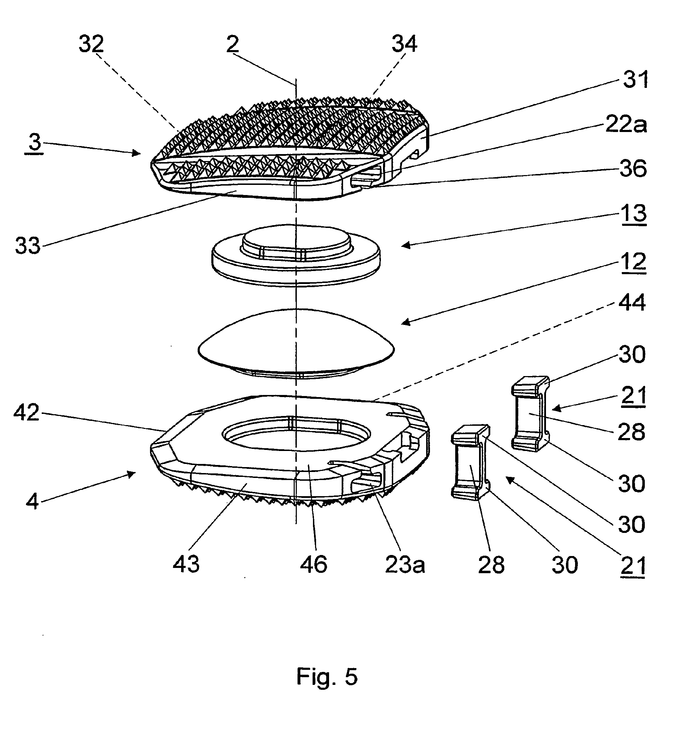

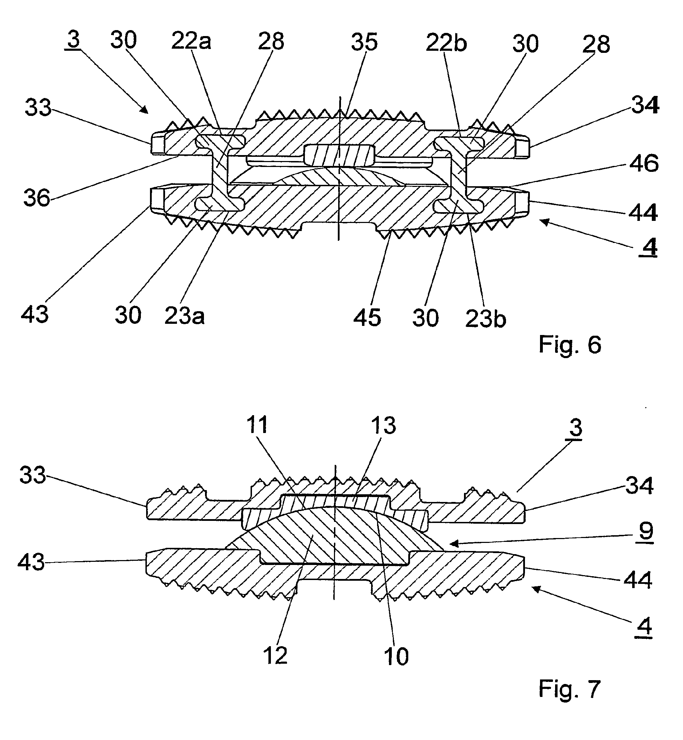

Die in den Fig. 4 bis 7 dargestellte Ausführungsform unterscheidet sich von der in den Fig. 1 bis 3 dargestellten Ausführungsform nur durch eine anderen Ausgestaltung der Nuten 22;23 und der Blockiermittel 21. Die Nuten 22;23 sind gegen die obere, respektive die untere Oberfläche 36;46 der zwei Teile 3;4 offen. Zudem sind die Nuten 22;23 gegen die jeweilige obere, respektive untere Oberfläche 36;46 verjüngt, d.h. beispielsweise als Schwalbenschwanzführungen ausgestaltet. Ferner sind die zwei Blockiermittel 21 je doppelt T-förmig ausgestaltet und umfassen einen Verbindungssteg 28 sowie endständig an diesem je einen Quersteg 30. Dabei ist je ein Blockiermittel 21 auf je einer Seite der Mittelebene 19 in die Nuten 22;23 einführbar. Der Querschnitt der Nuten 22;23 verringert sich mit zunehmender Distanz von den ventralen Seitenflächen 31;41, so dass sich die Blockiermittel 21 beim Einführung in die Nuten 22;23 in diesen verkeilen und lösbar an den zwei Teilen 3;4 befestigt sind.

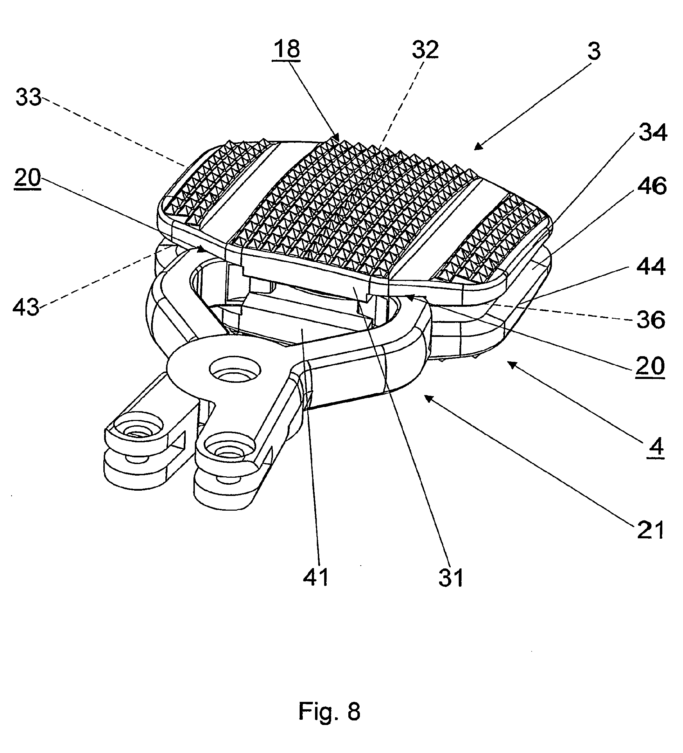

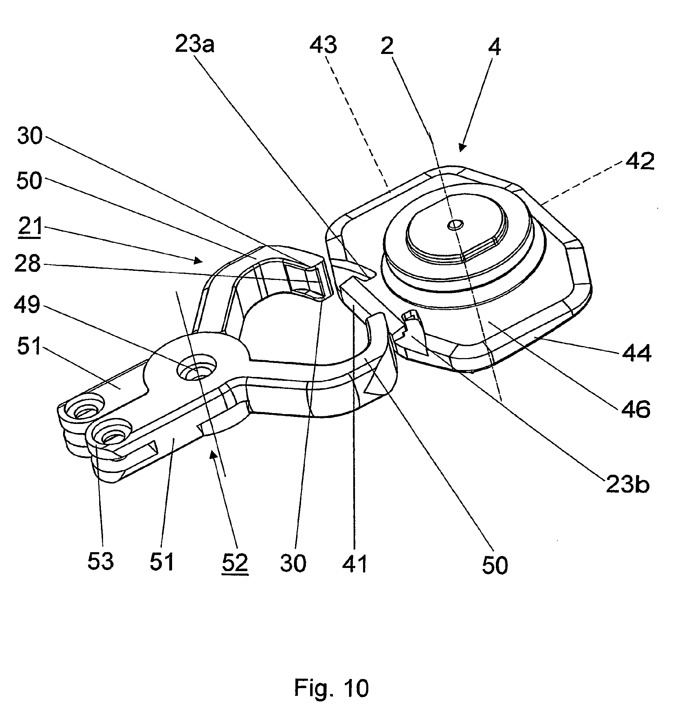

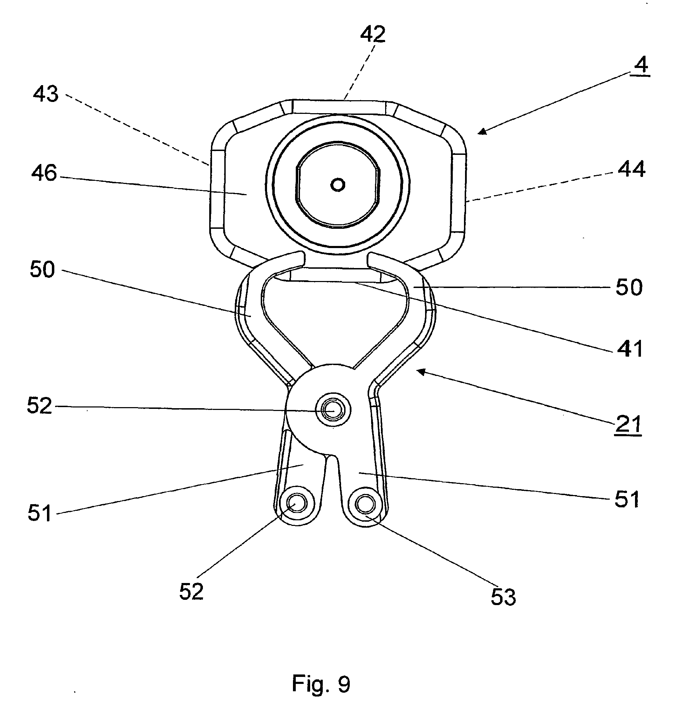

In den Fig. 8 bis 10 ist eine Ausführungsform dargestellt, welche sich von der in den Fig. 1 bis 7 nur durch eine andere Ausgestaltung der Nuten 22;23 und der Blockiermittel 21 unterscheidet. Die Nuten 22;23 sind gegen die obere, respektive untere Oberfläche 36;46 der zwei Teile 3;4 offen und weisen gekrümmte Längsachsen 24;25 auf. Die Krümmung der Nuten 22;23 ist derart ausgestaltet, dass der Abstand zwischen zwei an einem der Teile 3;4 angebrachten Nuten 22;23 gegen die ventrale Seitenfläche 31;41 zunimmt. Ferner sind die Nuten 22;23 gegen die jeweilige obere, respektive untere Oberfläche 36;46 verjüngt. Die Blockiermittel 21 umfassen hier zwei in die gekrümmten Nuten 22;23 einführbare Backen 50 mit Hebeln 51, welche durch ein Drehgelenk 52 relativ zueinander bewegbar verbunden sind. Die Drehachse 49 des Drehgelenkes 52 steht parallel zur Zentralachse 2. Die Backen 50 weisen eine U-förmige Querschnittsfläche auf und umfassen je einen zur Zentralachse 2 parallelen Verbindungssteg 28 und daran endständig je zwei Querstege 30, so dass bei in die Nuten 22;23 eingeführten Blockiermitteln 21 die zwei Teile 3;4 auch parallel zur Zentralachse 2 fixiert sind. An den freien Enden 53 der Hebel 51 ist ein Instrument befestigbar, womit das Zwischenwirbelimplantat 1 in den Zwischenwirbelraum einführbar ist und nach der Implantation die Blockiermittel 21 einfach aus den Nuten 22;23 herausziehbar sind.

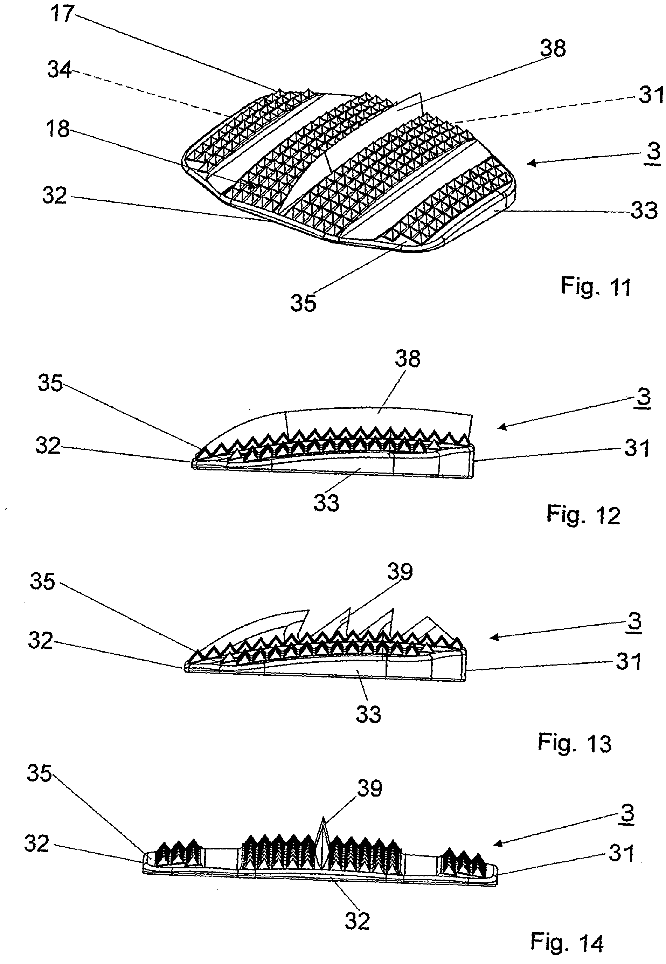

Neben den in den Fig. 1 bis 10 dargestellten pyramidenartigen Erhebungen 17 können die Erhebungen 18 auf der oberen und auf der unteren Appositionsfläche 35;45 wie in den Fig. 11 und 12 dargestellt, je eine keilförmige, gegenüber den pyramidenartigen Erhebungen 17 erhöhte Rippe 38 umfassen, welche parallel zu einer in der Mittelebene 19 (Fig. 1) liegenden Geraden auf den Appositionsflächen 35;45 steht. Damit das Zwischenwirbelimplantat 1 besser in den Zwischenwirbelraum einführbar ist, verringert sich die Höhe der Rippen 38 gegen die dorsale Seitenfläche 32.

Eine weitere Ausführungsform von Erhebungen 18 ist in den Fig. 13 und 14 dargestellt. Die gegenüber den pyramidenförmigen Erhebungen 17 erhöhten Zacken 39 sind teilweise sägezahnförmig ausgestaltet, wobei die steilere Flanke gegen die ventrale Seitenfläche 31 gerichtet ist. Die Zacken 39 liegen hier auf einer in der Mittelebene 19 (Fig. 1) liegenden Geraden und reichen von der ventralen Seitenfläche 31 bis zur dorsalen Seitenfläche 32.

In den Fig. 11 bis 14 ist jeweils nur das obere Teil 3 beispielhaft dargestellt. Dieselbe Ausgestaltung der Erhebungen 18 mit pyramidenförmigen Erhebungen 17, einer Rippe 38 (Fig. 11 und 12) oder Zacken 39 (Fig. 13 und 14) ist analog auf das untere Teil 4 übertragbar.

### Beschreibung der Operationstecknik:

Der Chirurg stellt die Bandscheibe von anterior durch einen transperitonealen oder retroperitonealen Zugang vor. Die Entfernung der Bandscheibe (Discectomie) nimmt er soweit vor, wie es die Breite des Implantates erfordert. Mittels einem geeigneten Instrument kann der Chirurg den Zwischenwirbelraum distrahieren, um mit dem später eingesetzten Zwischenwirbelimplantat eine Entlastung (Dekompression) zu erzeugen. Mit Hilfe eines Probeimplantates kann der Operateur die Grösse des Implantates festlegen. Der Distraktor bleibt immer noch im Einsatz.

Der Chirurg kann nun das Zwischenwirbelimplantat mittels einem geeigneten Instrument und den temporären Blockiermitteln in den präparierten Bandscheibenraum einsetzen. Währenddessen alle Instrumente noch anliegen, muss die endgültige Position des Implantates durch Schlagen und Schieben mittels der dafür vorgesehenen Instrumente gefunden werden. Damit das Implantat die funktionale Mobilität erhält, müssen anschliessend die temporären Blockiermittel und die zugehörenden Instrumente entfernt werden. Zum Schluss kann der Chirurg den Distraktor herausziehen und den Wundverschluss vornehmen.

## Patentansprüche

1. Zwischenwirbelimplantat (1), insbesondere künstliche Bandscheibe, mit einer Zentralachse (2), einem oberen Teil (3), einem unteren Teil (4) und einem axial zwischen den zwei Teilen (3;4) angeordneten Gelenk (9), wobei
A) das obere Teil (3) eine obere Appositionsfläche (35), eine ventrale Seitenfläche (31), eine dorsale Seitenfläche (32) und zwei laterale Seitenflächen (33;34) aufweist und die obere Appositionsfläche (35) zur Anlage an die Grundplatte eines darüber liegenden Wirbelkörpers geeignet ist;
B) das untere Teil (4) eine untere Appositionsfläche (45), eine ventrale Seitenfläche (41), eine dorsale Seitenfläche (42) und zwei laterale Seitenflächen (43;44) aufweist, und die untere Appositionsfläche (45) zur Anlage an die Deckplatte eines darunter liegenden Wirbelkörpers geeignet ist; und
C) das Gelenk (9) ein konvexes Gelenkteil (12) mit einer ersten Gelenkfläche (10) und eine dazu komplementäre Gelenkschale (13) mit einer auf der ersten Gelenkfläche (10) gleitbar gelagerten, zweiten Gelenkfläche (11) umfasst, und
D) jedes der zwei Teile (3;4) Mittel (20) zur Aufnahme von temporär befestigbaren Blockiermitteln (21) für die temporäre Fixation der zwei Teile (3;4) relativ zueinander umfasst;
**dadurch gekennzeichnet, dass**
E) das Zwischenwirbelimplantat (1) mindestens ein in die Mittel (20) eingeführtes temporäres Blockiermittel (21) umfasst, welches geeignet ist zusammen mit dem Zwischenwirbelimplantat (1) mittels eines geeigneten Instruments in den Zwischenwirbelraum eingeführt zu werden und nach erfolgter Implantation des blockierten Zwischenwirbelimplantats (1) wieder entfernt zu werden, so dass das implantierte Zwischenwirbelimplantat (1) seine funktionale Mobilität erhält; und
F) die Mittel (20) mindestens eine obere Nute (22) am oberen Teil (3) sowie mindestens eine untere Nute (23) am unteren Teil (4) umfassen, wobei die Längsachsen der Nuten (22;23) die ventralen Seitenflächen (31;41) des jeweiligen Teiles (3;4) schneiden; wobei
G) das Zwischenwirbelimplantat (1) mittig zwischen den lateralen Seitenflächen (33;34;43;44) eine die Zentralachse (2) enthaltende Mittelebene (19) aufweist und auf jeder Seite der Mittelebene (19) eine obere Nute (22a;22b) und eine untere Nute (23a;23b) umfasst.

2. Zwischenwirbelimplantat (1) nach Anspruch 1, **dadurch gekennzeichnet, dass** die Blockiermittel (21) einstückig sind.

3. Zwischenwirbelimplantat (1) nach Anspruch 1 oder 2, **dadurch gekennzeichnet, dass** die Blockiermittel (21) eine selbsttätige Fixation der zwei Teile (3;4) gestatten.

4. Zwischenwirbelimplantat (1) nach einem der Ansprüche 1-3, **dadurch gekennzeichnet, dass** die Nuten (22;23) in die ventrale Seitenfläche (31;41) des jeweiligen Teiles (3;4) münden.

5. Zwischenwirbelmplantat (1) nach einem der Ansprüche 1-4, **dadurch gekennzeichnet, dass** sich die quer zur Mittelebene (19) stehende Querschnittsfläche der Nuten (22;23) gegen die obere, respektive untere Oberfläche (36;46) der zwei Teile (3;4) verjüngt.

6. Zwischenwirbelimplantat (1) nach einem der Ansprüche 1-5, **dadurch gekennzeichnet, dass** die Nuten (22;23) gekrümmt sind und bogenförmige Längsachsen aufweisen, wobei die Abstände der Längsachsen zur Mittelebene (19) gegen die ventrale Seitenflächen (31;41) zunehmen.

7. Zwischenwirbelimplantat (1) nach einem der Ansprüche 1 bis 4, **dadurch gekennzeichnet, dass** die Blockiermittel (21) einen zur Zentralachse (2) parallel angeordneten Verbindungssteg (28) und endständig an diesem je einen Schenkel (27) oder Quersteg (30) umfassen.

8. Zwischenwirbelimplantat (1) nach Anspruch 6, **dadurch gekennzeichnet, dass** die Blockiermittel (21) zangenartig ausgestaltet sind und zwei um ein Drehgelenk (52) relativ zueinander bewegbare, in die Nuten 22;23 einführbare Backen (50) umfassen.

9. Zwischenwirbelimplantat (1) nach Anspruch 8, **dadurch gekennzeichnet, dass** die Drehachse (49) des Drehgelenkes (52) parallel zur Zentralachse (2) verläuft.

10. Zwischenwirbelimplantat (1) nach einem der Ansprüche 1 bis 9, **dadurch gekennzeichnet, dass** das konvexe Gelenkteil (12) sphärisch ausgestaltet ist.

11. Zwischenwirbelimplantat (1) nach Anspruch 10, **dadurch gekennzeichnet, dass** der Radius der ersten Gelenkfläche (10) des konvexen Gelenkteiles (12) zwischen 3 mm und 25 mm beträgt.

12. Zwischenwirbelimplantat (1) nach Anspruch 11, **dadurch gekennzeichnet, dass** der Radius der ersten Gelenkfläche (10) des konvexen Gelenkteiles (12) zwischen 4 mm und 20 mm beträgt.

13. Zwischenwirbelimplantat (1) nach einem der Ansprüche 1 bis 12, **dadurch gekennzeichnet, dass** das konvexe Gelenkteil (12) und die Gelenkschale (13) aus einer Metall - Kunststoff Materialpaarung bestehen.

14. Zwischenwirbelimplantat (1) nach einem der Ansprüche 1 bis 13, **dadurch gekennzeichnet, dass** die Gelenkflächen (10;11) mit Titankarbid oder amorphem Kohlenstoff (ADLC) beschichtet sind.

15. Zwischenwirbelimplantat (1) nach einem der Ansprüche 1 bis 14, **dadurch gekennzeichnet, dass** die zwei Teile (3;4) an den Appositionsflächen (5;7) mit Titan beschichtet sind.

16. Zwischenwirbelimplantat (1) nach einem der Ansprüche 1 bis 15, **dadurch gekennzeichnet, dass** die Appositionflächen (5;7) konvex ausgebildet sind.

17. Zwischenwirbelimplantat (1) nach einem der Ansprüche 1 bis 16, **dadurch gekennzeichnet, dass** die zwei Teile (3;4) an ihren Appositionsflächen (5;7) mindestens teilweise mit makroskopischen Strukturen versehen sind.

18. Zwischenwirbelimplantat (1) nach Anspruch 17, **dadurch gekennzeichnet, dass** die makroskopischen Strukturen Erhebungen (18) sind.

19. Zwischenwirbelimplantat (1) nach Anspruch 18, **dadurch gekennzeichnet, dass** die Erhebungen (18) pyramidenartige Erhebungen (17) umfassen.

20. Zwischenwirbelimplantat (1) nach Anspruch 18 oder 19, **dadurch gekennzeichnet, dass** die Erhebungen (18) mindestens eine zur Mittelebene (19) symmetrische, keilartige Rippe (38) umfassen, welche parallel zu einer in der Mittelebene (19) liegenden Geraden auf der betreffenden Appositionsfläche 31 ;41 steht.

21. Zwischenwirbelimplantat (1) nach Anspruch 18 oder 19, **dadurch gekennzeichnet, dass** die Erhebungen (18) zur Mittelebene (19) symmetrisch angeordnete, sägezahnartige Zacken (39) umfassen.

22. Zwischenwirbelimplantat (1) nach einem der Ansprüche 19 bis 21, **dadurch gekennzeichnet, dass** eine pyramidenartige Erhebung (17) ein Volumen V zwischen 0,12 mm³ und 1,4 mm³ aufweist.

23. Zwischenwirbelimplantat (1) nach einem der Ansprüche 18 bis 22, **dadurch gekennzeichnet, dass** die Erhebungen (18) mindestens teilweise mit Hydroxylapatit oder mit einem bi-phasischen Hydroylapatit-Trikalziumphosphat Gemisch beschichtet ist.

24. Zwischenwirbelimplantat (1) nach einem der Ansprüche 1 bis 23, **dadurch gekennzeichnet, dass** die Blockiermittel (21) ein maximales Volumen von 12 cm³ aufweisen.

25. Zwischenwirbelimplantat (1) nach Anspruch 24, **dadurch gekennzeichnet, dass** die Blockiermittel (21) ein maximales Volumen von 6 cm³ aufweisen.

26. Zwischenwirbelimplantat (1) nach einem der Ansprüche 1 bis 25, **dadurch gekennzeichnet, dass** es eine sterile Verpackung umfasst.

## Claims

1. An intervertebral implant (1), in particular an artificial intervertebral disk, with a central axis (2), a top part (3), a bottom part (4) and a joint (9) arranged axially between the two parts (3, 4), wherein
A) the top part (3) has a top apposed surface (35), a ventral side surface (31), a dorsal side surface (32) and two lateral side surfaces (33, 34) and the top apposed surface (35) is suitable to be placed on the base plate of a body of the vertebra adjacent above,
B) the bottom part (4) has a bottom apposed surface (45), a ventral side surface (41), a dorsal side surface (42) and two lateral side surfaces (43, 44) and the bottom apposed surface (45) is suitable to be placed on the cover plate of a body of the vertebra adjacent below,
C) the joint (9) comprises a convex joint part (12) with a first articular surface (10) and a joint shell (13) matching it, with a second articular surface (11) mounted in a sliding manner on the first articular surface (10), and
D) each of the two parts (3, 4) comprises means (20) to accommodate temporarily fastenable locking means (21) to temporarily fix the two parts (3, 4) relative one another,
**characterised in that**
E) the intervertebral implant (1) comprises at least one temporary locking means (21) inserted into the means (20), which is suitable to be introduced into the intervertebral space together with the intervertebral implant (1) by means of a suitable instrument and to be removed after the implantation of the locked intervertebral implant (1) has been effected, such that the implanted intervertebral implant (1) receives its functional mobility;
F) that the means (20) comprise at least one top groove (22) on the top part (3) as well as at least one bottom groove (23) on the bottom part (4), while the longitudinal axes of the grooves (22, 23) intersect the ventral side surfaces (31, 41) of the respective part (3, 4); and
G) the intervertebral implant (1) has in the middle between the lateral side surfaces (33, 34, 43, 44) a central plane (19) that contains the central axis (2) and comprises a top groove (22a, 22b) and a bottom groove (23a, 23b) on each side of the central plane (19).

2. An intervertebral implant (1) according to claim 1, **characterised in that** the locking means (21) is made from one piece.

3. An intervertebral implant (1) according to claim 1 or 2, **characterised in that** the locking means (21) allow an automatic fixing of the two parts (3, 4).

4. An intervertebral implant (1) according to one of the claims 1 to 3, **characterised in that** the grooves (22, 23) terminate in the ventral side surface (31, 41) of the respective part (3, 4).

5. An intervertebral implant (1) according to one of the claims 1 to 4, **characterised in that** the cross-section of the grooves (22, 23) situated perpendicularly to the central plane (19) narrows towards the top and bottom surfaces (36, 46), respectively, of the two parts (3, 4).

6. An intervertebral implant (1) according to one of the claims 1 to 5, **characterised in that** the grooves (22, 23) are curved and have arch-shaped longitudinal axes, while the distances of the longitudinal axes towards the central plane (19) increase relative to the ventral side.

7. An intervertebral implant (1) according to any one of claims 1 to 4, **characterised in that** the locking means (21) comprise a joining web (28) that is arranged parallel to the central axis (2) and at the end of it a leg (27) or a transverse web (30) each.

8. An intervertebral implant (1) according to claim 6, **characterised in that** the locking means (21) have a pliers-like construction and comprise two jaws (50) which can move relative one another about a pivot hinge (52) and can be introduced into the grooves (22, 23).

9. An intervertebral implant (1) according to claim 8, **characterised in that** the axis (49) of rotation of the pivot hinge (52) extends parallel to the central axis (2).

10. An intervertebral implant (1) according to one of the claims 1 to 9, **characterised in that** the convex joint part (12) has a spherical construction.

11. An intervertebral implant (1) according to claim 10, **characterised in that** the radius of the first articular surface (10) of the convex spherical joint (12) part is between 3 mm and 25 mm.

12. An intervertebral implant (1) according to claim 11, **characterised in that** the radius of the first articular surface (10) of the convex spherical joint (12) part is between 4 mm and 20 mm.

13. An intervertebral implant (1) according to one of the claims 1 to 12, **characterised in that** the convex joint part (12) and the joint shell (13) are made from a metal/plastics material pair.

14. An intervertebral implant (1) according to one of the claims 1 to 13, **characterised in that** the articular surfaces (10, 11) are coated with titanium carbide or amorphous carbon (ADLC).

15. An intervertebral implant (1) according to one of the claims 1 to 14, **characterised in that** the two parts (3, 4) are coated with titanium on the apposed surfaces (5, 7).

16. An intervertebral implant (1) according to one of the claims 1 to 15, **characterised in that** the apposed surfaces (5, 7) have a convex construction.

17. An intervertebral implant (1) according to one of the claims 1 to 16, **characterised in that** the apposed surfaces (5, 7) of the two parts (3, 4) are provided at least partially with macroscopic structures.

18. An intervertebral implant (1) according to claim 17, **characterised in that** the macroscopic structures are protuberances (18).

19. An intervertebral implant (1) according to claim 18, **characterised in that** the protuberances (18) comprise pyramid-like protuberances (17)

20. An intervertebral implant (1) according to claim 18 or 19, **characterised in that** the protuberances (18) comprise at least one wedge-shaped rib (38) that is symmetrical about the central plane (19) and is situated on the respective apposed surfaces (31, 41) in a straight line that is parallel to the central plane (19).

21. An intervertebral implant (1) according to claim 18 or 19, **characterised in that** the protuberances (18) comprises saw-tooth like serrations (39) arranged symmetrically about the central plane (19).

22. An intervertebral implant (1) according to one of the claims 19 to 21, **characterised in that** a pyramid-shaped protuberance (17) has a volume V between 0.12 mm³ and 1.4 mm³.

23. An intervertebral implant (1) according to one of the claims 18 to 22, **characterised in that** the protuberances (18) are coated at least partially with hydroxylapatite or with a bi-phased hydroxylapatite-tricalcium phosphate mixture.

24. An intervertebral implant (1) according to one of the claims 1 to 23, **characterised in that** the locking means (21) have a maximum volume of 12cm³.

25. An intervertebral implant (1) according to claim 24, **characterised in that** the locking means (21) have a maximum volume of 6 cm³.

26. An intervertebral implant (1) according to one of the claims 1 to 25, **characterised in that** it comprises a sterile packaging.

## Revendications

1. Implant intervertébral (1), notamment disque intervertébral artificiel, comprenant un axe central (2), une pièce supérieure (3), une pièce inférieure (4) et une articulation (9) disposée axialement entre les deux pièces (3 ; 4),
A) la pièce supérieure (3) présentant une face d'apposition supérieure (35), une face ventrale (31), une face dorsale (32) et deux faces latérales (33 ; 34) et la face d'apposition supérieure (35) étant appropriée pour porter contre la plaque inférieure d'un corps vertébral sus-jacent ;
B) la pièce inférieure (4) présentant une face d'apposition inférieure (45), une face ventrale (41), une face dorsale (42) et deux faces latérales (43 ; 44) et la face d'apposition inférieure (45) étant appropriée pour porter contre la plaque supérieure d'un corps vertébral sous-jacent ;
C) l'articulation (9) comportant une pièce d'articulation convexe (12) présentant une première face d'articulation (10) et une coque d'articulation (13), complémentaire de ladite pièce d'articulation, présentant une deuxième face d'articulation (11) montée, de façon à pouvoir glisser, sur la première face d'articulation (10), et
D) chacune des deux pièces (3 ; 4) comportant des moyens (20) de réception de moyens de blocage (21) pouvant être temporairement fixés et destinés à la fixation temporaire des deux pièces (3 ; 4) l'une par rapport à l'autre ;
**caractérisé en ce que**
E) l'implant intervertébral (1) comporte au moins un moyen de blocage temporaire (21) qui est inséré dans les moyens (20) et qui est approprié pour être introduit dans l'espace intervertébral, conjointement avec l'implant intervertébral (1), au moyen d'un instrument approprié et pour être retiré après la mise en place de l'implant intervertébral bloqué (1) de façon à conférer à l'implant intervertébral (1) en place sa mobilité fonctionnelle ; et
F) les moyens (20) comportent au moins une gorge supérieure (22) ménagée au niveau de la pièce supérieure (3) ainsi qu'au moins une gorge inférieure (23) ménagée au niveau de la pièce inférieure (4), les axes longitudinaux des gorges (22 ; 23) coupant les faces ventrales (31 ; 41) de la pièce respective (3 ; 4) ;
G) l'implant intervertébral (1) comporte au milieu, entre les faces latérales (33 ; 34 ; 43 ; 44), un plan médian (19) qui contient l'axe central (2) et, de chaque côté du plan médian (19), une gorge supérieure (22a ; 22b) et une gorge inférieure (23a ; 23b).

2. Implant intervertébral (1) selon la revendication 1, **caractérisé en ce que** les moyens de blocage (21) sont d'une seule pièce.

3. Implant intervertébral (1) selon la revendication 1 ou 2, **caractérisé en ce que** les moyens de blocage (21) constituent une fixation automatique des deux pièces (3 ; 4).

4. Implant intervertébral (1) selon l'une des revendications 1 à 3, **caractérisé en ce que** les gorges (22 ; 23) débouchent dans la face ventrale (31 ; 41) de la pièce respective (3 ; 4).

5. Implant intervertébral (1) selon l'une des revendications 1 à 4, **caractérisé en ce que** la section des gorges (22 ; 23), qui est transversale au plan médian (19), s'amincit en direction de la surface supérieure, respectivement inférieure, (36 ; 46) des deux pièces (3 ; 4).

6. Implant intervertébral (1) selon l'une des revendications 1 à 5, **caractérisé en ce que** les gorges (22 ; 23) sont incurvées et comportent un axe longitudinal en forme d'arc, la distance des axes longitudinaux par rapport au plan médian (19) augmentant en direction des faces ventrales (31 ; 41).

7. Implant intervertébral (1) selon l'une des revendications 1 à 4, **caractérisé en ce que** les moyens de blocage (21) comportent une nervure de liaison (28) disposée parallèlement à l'axe central (2) et, à chaque extrémité de celle-ci, une branche (27) ou nervure transversale (30).

8. Implant intervertébral (1) selon la revendication 6, **caractérisé en ce que** les moyens de blocage (21) sont conformés en tenaille et comportent deux mâchoires (50) qui sont aptes à se déplacer l'une par rapport à l'autre autour d'une articulation rotative (52) et qui peuvent être introduites dans les gorges (22 ; 23).

9. Implant intervertébral (1) selon la revendication 8, **caractérisé en ce que** l'axe de rotation (49) de l'articulation rotative (52) s'étend parallèlement à l'axe central (2).

10. Implant intervertébral (1) selon l'une des revendications 1 à 9, **caractérisé en ce que** la pièce d'articulation convexe (12) a une conformation sphérique.

11. Implant intervertébral (1) selon la revendication 10, **caractérisé en ce que** le rayon de la première face d'articulation (10) de la pièce d'articulation convexe (12) est compris entre 3 mm et 25 mm.

12. Implant intervertébral (1) selon la revendication 11, **caractérisé en ce que** le rayon de la première face d'articulation (10) de la pièce d'articulation convexe (12) est compris entre 4 mm et 20 mm.

13. Implant intervertébral (1) selon l'une des revendications 1 à 12, **caractérisé en ce que** la pièce d'articulation convexe (12) et la coque d'articulation (13) sont constituées d'un appariement de matériaux métal/matière plastique.

14. Implant intervertébral (1) selon l'une des revendications 1 à 13, **caractérisé en ce que** les faces d'articulation (10 ; 11) sont recouvertes de carbure de titane ou de carbone amorphe (ADLC).

15. Implant intervertébral (1) selon l'une des revendications 1 à 14, **caractérisé en ce que** les deux pièces (3 ; 4) sont revêtues de titane sur les faces d'apposition (5 ; 7).

16. Implant intervertébral (1) selon l'une des revendications 1 à 15, **caractérisé en ce que** les faces d'apposition (5 ; 7) ont une conformation convexe.

17. Implant intervertébral (1) selon l'une des revendications 1 à 16, **caractérisé en ce que** les deux pièces (3 ; 4) sont dotées au moins partiellement de structures macroscopiques sur leurs faces d'apposition (5 ; 7).

18. Implant intervertébral (1) selon la revendication 17, **caractérisé en ce que** les structures macroscopiques sont des éminences (18).

19. Implant intervertébral (1) selon la revendication 18, **caractérisé en ce que** les éminences (18) comportent des éminences pyramidales (17).

20. Implant intervertébral (1) selon la revendication 18 ou 19, **caractérisé en ce que** les éminences (18) comportent au moins une arête cunéiforme (38) qui est symétrique par rapport au plan médian (19) et qui s'étend sur ladite surface d'apposition (31 ; 41) parallèlement à une droite se trouvant dans le plan médian (19).

21. Implant intervertébral (1) selon la revendication 18 ou 19, **caractérisé en ce que** les éminences (18) comportent des pointes (39) en dents de scie qui sont disposées symétriquement par rapport au plan médian (19).

22. Implant intervertébral (1) selon l'une des revendications 19 à 21, **caractérisé en ce qu'**une éminence pyramidale (17) présente un volume V compris entre 0,12 mm³ et 1,4 mm³.

23. Implant intervertébral (1) selon l'une des revendications 18 à 22, **caractérisé en ce que** les éminences (18) sont recouvertes au moins partiellement d'hydroxylapatite ou d'un mélange biphasique hydroxylapatite/phosphate tricalcique.

24. Implant intervertébral (1) selon l'une des revendications 1 à 23, **caractérisé en ce que** les moyens de blocage (21) ont un volume maximum de 12 cm³.

25. Implant intervertébral (1) selon la revendication 24, **caractérisé en ce que** les moyens de blocage (21) ont un volume maximum de 6 cm³.

26. Implant intervertébral (1) selon l'une des revendications 1 à 25, **caractérisé en ce qu'**il comporte un emballage stérile.
